# EUROPEAN PATENT APPLICATION

(11) **EP 3 150 181 A1**
(43) Date of publication of application: **05.04.2017**
(21) Application number: 16191369.4
(22) Date of filing: 29.09.2016
(51) Int. Cl.: A61F 9/007, A61M 1/00, F04B 43/12

(54) **OPHTHALMIC SURGICAL APPARATUS**

(30) Priority: 30.09.2015 JP 2015194772
(71) Applicant: Nidek Co., Ltd., Gamagori-shi, Aichi 443-0038 (JP)
(72) Inventor: KATO, Hisatoshi, Gamagori-shi, Aichi 443-0038 (JP)
(74) Representative: Prüfer & Partner mbB Patentanwälte · Rechtsanwälte

(57) **Abstract**

An ophthalmic surgical apparatus (1) supplies perfusion solution into a patient's eye (E) from a perfusion solution container (17) stored with the perfusion solution. The ophthalmic surgical apparatus (1) includes a perfusion passage (5), a non-positive displacement pump (50), a perfusion pressurizing motor (51), and a controller (40). The perfusion passage (5) introduces the perfusion solution in the perfusion solution container (17) to a surgical instrument which will be inserted in an eye. The non-positive displacement pump (50) provided in the perfusion passage (5) includes a blade-like rotor which is rotatable without shutting off the perfusion passage (5). The perfusion pressurizing motor (51) applies pressure to the perfusion solution by rotating the rotor. The controller (40) controls the drive to the perfusion pressurizing motor (51).

## Description

### BACKGROUND

The present disclosure relates to an ophthalmic surgical apparatus to supply perfusion solution into a patient's eye and then perform a surgery to the patient's eye.

An ophthalmic surgical apparatus for an ophthalmic surgery (such as a cataract surgery and a vitreous surgery) has been known. In one example, the ophthalmic surgical apparatus is configured to supply perfusion solution into an eye via a surgical instrument which is connected to the surgical apparatus. The ophthalmic surgical apparatus carries out a surgery for the patient's eye by aspirating an eliminated tissue with the perfusion solution which has been supplied into the eye.

In some ophthalmic surgeries, perfusion solution which is to be supplied into an eye is adjusted its pressure (hereinafter, called as "perfusion pressure"). Various methods have been proposed for adjusting or controlling the perfusion pressure. One example of the methods is to change height of a container stored with the perfusion solution (hereinafter, called as a "perfusion solution container") so as to control the perfusion pressure. In this method, however, it is difficult in most cases to quickly change the height of the perfusion solution container, and hence speed (responsivity) to change the perfusion pressure is rarely improved.

There is another method of the perfusion pressure control to apply pressure to the perfusion solution without changing the height of the perfusion solution container. An apparatus described in Japanese Patent Application Publication No. 04-504514 (Patent Document 1) is, for example, configured to feed pressurizing gas into a liquid-injection container having a limited capacity and thereby apply pressure to the perfusion solution in the container. As another apparatus, a pressure-type infusion device described in Japanese Patent Application Publication No. 2012-527307 (Patent Document 2) is configured such that a bag stored with solution is placed between a flexible band and a base. Operation of a motor applies a tensile force to the band, thereby changing pressure inside the bag.

### SUMMARY

The conventional methods of applying pressure to the perfusion solution have some deficiencies. One of such deficiencies is that a type and others of the perfusion solution container is limited in the methods disclosed in Patent Documents 1 and 2. Specifically, the method of Patent Document 1 requires a container which is hard to be deformed and hence a capacity of the container is limited. The method of Patent Document 2 requires a specialized bag which is allowed to be compressed. Further, the conventional methods have another problem that an apparatus configuration and others become complicated.

A typical object of the present disclosure is to provide an ophthalmic surgical apparatus enabling to appropriately supply perfusion solution into an eye.

An ophthalmic surgical apparatus provided in typical embodiments of the present disclosure is An ophthalmic surgical apparatus configured to supply perfusion solution into a patient's eye from a perfusion solution container stored with the perfusion solution, the ophthalmic surgical apparatus comprising: a perfusion passage configured to introduce the perfusion solution in the perfusion solution container to a surgical instrument which will be inserted in the eye; a non-positive displacement pump provided in the perfusion passage, the non-positive displacement pump including a blade-like rotor which is rotatable without shutting off the perfusion passage; a motor configured to rotate the rotor and thus to apply pressure to the perfusion solution; and a controller configured to control the drive to the motor. Further developments of the present disclosure are given in the dependent claims.

### BRIEF DESCRIPTION OF THE DRAWINGS

FIG. 1 is an external perspective view of an ophthalmic surgical apparatus;
FIG. 2 is a schematic diagram showing a configuration of the ophthalmic surgical apparatus;
FIG. 3 is an exploded perspective view of a non-positive displacement pump and a perfusion pressurizing motor;
FIG. 4 is a plan view of the non-positive displacement pump from which a cover is removed; and
FIG. 5 is a flow chart of a perfusion solution supply process carried out by the ophthalmic surgical apparatus.

### DETAILED DESCRIPTION OF THE EXEMPLARY EMBODIMENTS

One typical embodiment of the present disclosure is now explained below in detail with reference to the accompanying drawings. The present embodiment is explained with illustrating an ophthalmic surgical apparatus 1 which is enabled to perform both a cataract surgery and a vitreous surgery for a patient's eye E. The technique illustrated in the present disclosure may also be embodied in an ophthalmic surgical apparatus performing only a cataract surgery and an ophthalmic surgical apparatus performing only a vitreous surgery, for example.

### <Overall configuration>

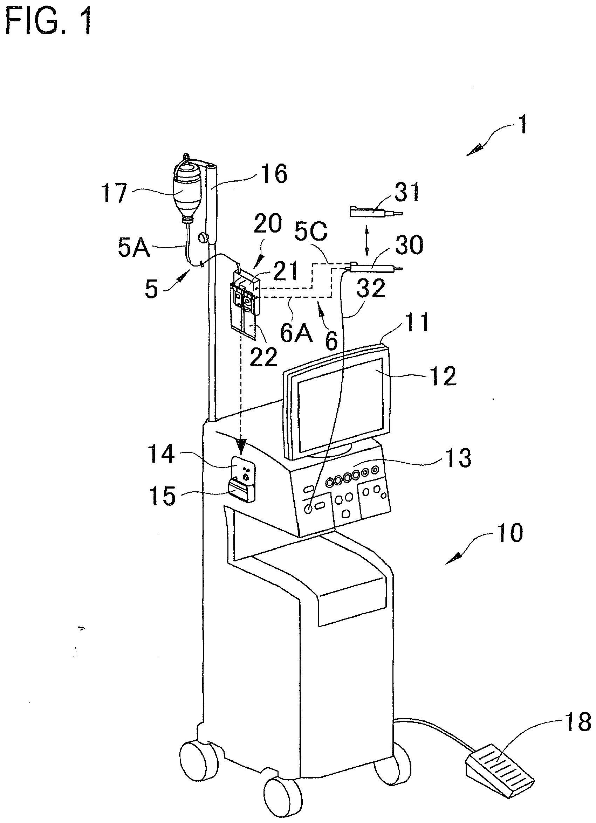

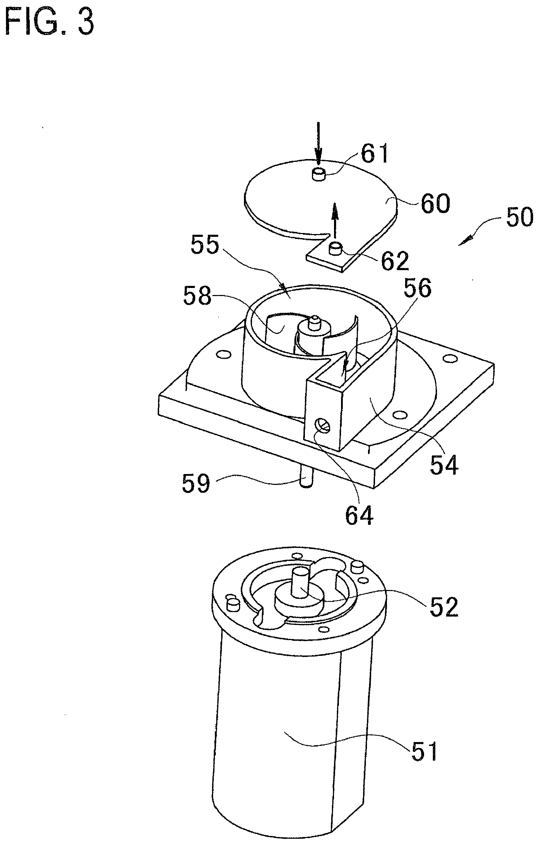

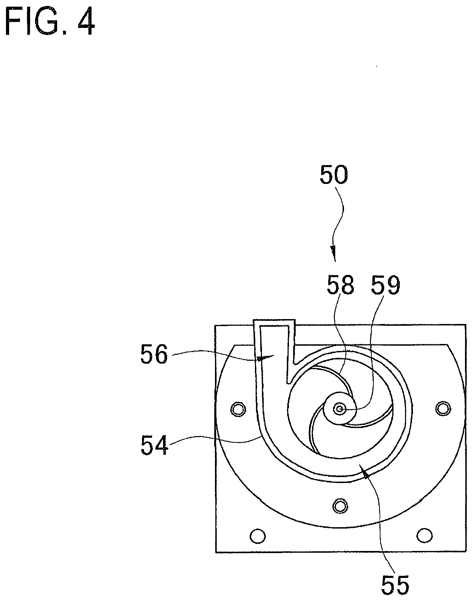

An overall configuration of the ophthalmic surgical apparatus 1 of the present embodiment is explained with reference to FIG. 1. The ophthalmic surgical apparatus 1 of the present embodiment is provided with an apparatus body 10 and a cassette 20. The apparatus body 10 includes various configurations necessary for performing ophthalmic surgery in its housing. The cassette 20 is a disposable component provided with various configurations which are required to be kept in a clean state, and this cassette 20 is attached to the apparatus body 10 in a detachable manner. The cassette 20 includes a cassette body 21 provided with a non-positive displacement pump 50 (see FIGs. 3 and 4) and others and a waste fluid container (for example, a waste fluid bag) 22 in which waste fluid aspirated from the patient's eye E is stored.

The apparatus body 10 is provided with a monitor 11, a touch screen 12, a connection panel 13, a cassette holding unit 14, a container holding unit 16, and a foot switch 18.

The monitor 11 is provided on a front side in an upper part of the apparatus body 10 to display various images. The monitor 11 may be displayed with a screen for setting surgical conditions. The surgical conditions include selection of a surgery mode by a user, setting the height of a perfusion solution container 17, setting function of the foot switch 18, setting perfusion pressure, setting aspiration pressure, setting intraocular pressure, and setting ultrasonic power, and these conditions may be set or determined on the setting screen. The touch screen 12 is formed on a surface of a display screen of the monitor 11 to receive various operation commands input by the user.

The connection panel 13 is provided with at least one connector to which a cable or the like of a surgical instrument is connected. The surgical instrument is operated by the user (an operator) to be in contact with or adjacent to the patient's eye E (in the present embodiment, inserted into the patient's eye E). The connection panel 13 of the present embodiment is formed on a front surface of the housing of the apparatus body 10. One example of the surgical instrument used in the present embodiment is a US handpiece (ultrasonic handpiece) 30. The US handpiece 30 is configured to emulsify a nucleus of lens, which has become opaque due to cataract, by ultrasonic oscillation of a fragmentation tip provided on a tip end of the handpiece and then remove the nucleus of lens by suction. The US handpiece 30 is provided with an ultrasonic oscillator and this ultrasonic oscillator may be supplied with electric power through a power cable 32 to generate ultrasonic oscillation on the fragmentation tip. Another surgical instrument other than the US handpiece 30 may be utilized. For instance, an I/A handpiece (irrigation aspiration handpiece) 31 may be utilized as the surgical instrument. As further alternative, a surgical instrument for supplying perfusion solution into an eye during a vitreous surgery may also be employed.

The cassette holding unit 14 is attached with the cassette 20 in a detachable manner. The cassette holding unit 14 of the present embodiment is provided on a side surface of the housing of the apparatus body 10. In the present embodiment, the cover 15 is pushed toward the housing when the cassette 20 is placed in a predetermined position of the cassette holding unit 14, and thus the cassette 20 is attached to (loaded with) the cassette holding unit 14. The apparatus body 10 may be provided with a plurality of cassette holding units 14.

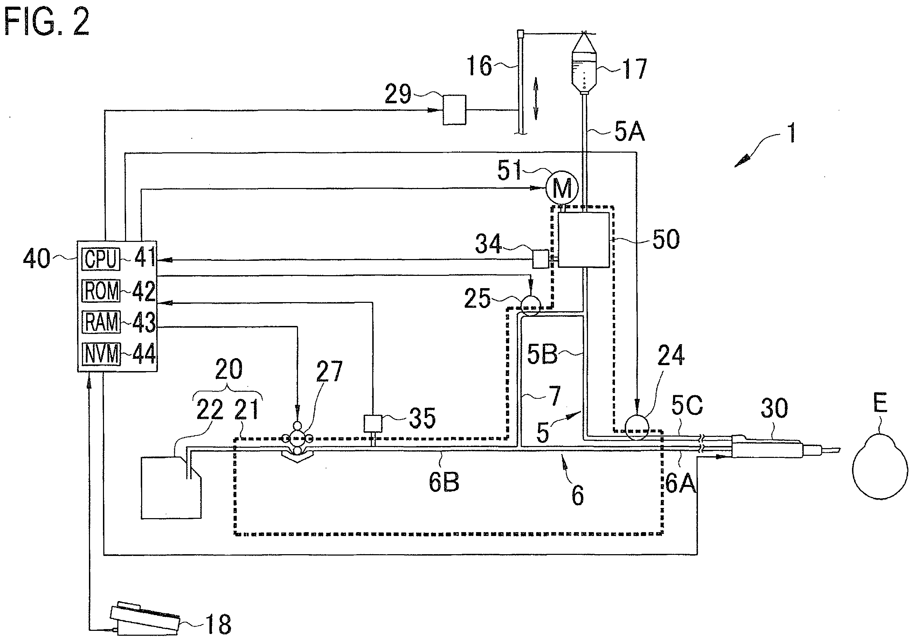

The container holding unit 16 holds the perfusion solution container 17 stored with the perfusion solution. The container holding unit 16 of the present embodiment is able to hold the container 17 in a position equal to or higher than the height of the patient's eye E. The container holding unit 16 of the present embodiment is further able to hold the container 17 in a position equal to or higher than the non-positive displacement pump 50 (see FIGs. 3 and 4) which will be mentioned later. As one example, the container holding unit 16 is provided with a pole and an arm. The pole extends upward from the apparatus body 10. The arm extends sideways from the pole to hang the container 17. The ophthalmic surgical apparatus 1 of the present embodiment is further provided with a pole vertical-motion motor 29 (see FIG. 2) and a vertical motion mechanism to change the height of the container 17 held by the container holding unit 16. Specifically, rotation of the pole vertical-motion motor 29 moves the container holding unit 16 upward and downward. The configuration of the container holding unit 16 and others may be modified. For example, the container holding unit 16 may be provided inside the housing of the apparatus body 10.

The perfusion solution container 17 is stored with the perfusion solution. As for the perfusion solution, physiological saline solution may be used, for example. Various configurations may be adopted for the perfusion solution container 17. The container 17 may be a deformable package made of soft material (such as vinyl), or may be a bottle made of material hard to be deformed (such as glass or hard plastic). Use of the technique illustrated in the present embodiment makes it possible to adopt a number of types of the containers 17, and it will be explained in detail below.

The perfusion solution container 17 and the surgical instrument are connected via a perfusion passage 5 (5A to 5C). The perfusion passage 5 introduces the perfusion solution inside the container 17 to the surgical instrument. As one example, the container 17 and the surgical instrument are connected via a perfusion passage (perfusion tube) 5A connecting the container 17 and the cassette 20, a perfusion passage 5B (see FIG. 2) provided inside the cassette 20, and a perfusion passage (perfusion tube) 5C connecting the cassette 20 and the surgical instrument in the present embodiment.

The surgical instrument and the waste fluid container 22 are connected via an aspiration passage 6 (6A and 6B). The aspiration passage 6 introduces the waste fluid (including an eliminated tissue and the perfusion solution) aspirated from the eye into the waste fluid container 22. As one example, the surgical instrument and the waste liquid container 22 are connected via an aspiration passage (aspiration tube) 6A connecting the surgical instrument with the cassette 20 and an aspiration passage 6B (see FIG. 2) provided in the cassette 20.

In an example illustrated in FIGs. 1 and 2, the perfusion passage 5 and the aspiration passage 6 are both connected to the same surgical instrument. Alternatively, the perfusion passage 5 and the aspiration passage 6 may be separately connected to different surgical instruments. Specifically, in a vitreous surgery, the perfusion passage 5 may be connected to a surgical instrument for supplying the perfusion solution into the eye and the aspiration passage 6 may be connected to a vitreous cutter.

The foot switch 18 is operated by the user to output signals corresponding to the subject operation to a controller 40 (see FIG. 2) of the ophthalmic surgical apparatus 1. The foot switch 18 may be operated for adjusting ultrasonic oscillation of a movable tip which is provided on a tip end of the US handpiece 30 or for controlling an aspiration operation for the eliminated tissue, and others.

### <Inner configuration>

A schematic configuration of the cassette 20 of the present embodiment and a connecting relation of the cassette 20 with each component of the apparatus body 10 (see FIG. 1) are explained with reference to FIG. 2. As mentioned above, the cassette body 21 of the cassette 20 is provided with the perfusion passage 5B and the aspiration passage 6B. The cassette body 21 of the present embodiment is further provided with a vent passage 7. The vent passage 7 is a fluid passage connecting the perfusion passage 5B and the aspiration passage 6B. The vent passage 7 branches off of the perfusion passage 5B between the non-positive displacement pump 50 and a perfusion valve 24 (which will be explained in detail below) in the present embodiment. The vent passage 7 branching off of the perfusion passage 5B is joined to the aspiration passage 6B between the surgical instrument and an aspiration pump 27 (which will be explained in detail below).

The perfusion solution is supplied from the perfusion solution container 17 into the eye through the surgical instrument, and then flows in the waste liquid container 22 from the surgical instrument. Accordingly, passages including the perfusion passage 5 and the aspiration passage 6 in which the perfusion solution flows are each defined such that a side closer to the container 17 is an upstream side and a side closer to the waste liquid container 22 is a downstream side. The perfusion passage 5A extending from the container 17 is connected to an upstream-side end portion of the perfusion passage 5B in the cassette body 21. The perfusion passage 5C connected to the surgical instrument is connected to a downstream-side end portion of the perfusion passage 5B in the cassette body 21.

The apparatus body 10 is provided with the perfusion valve 24 and a vent valve 25. The perfusion valve 24 is configured to open and close the perfusion passage 5 to switch supplying and shutting off flow of the perfusion solution to the surgical instrument through the perfusion passage 5. In the present embodiment, when the cassette 20 is attached to the apparatus body 10, the perfusion valve 24 is connected to the perfusion passage 5B in the cassette body 21. To be more specific, the perfusion valve 24 of the present embodiment is connected to the perfusion passage 5B on a downstream side of a portion where the vent passage 7 branches off. The vent valve 25 is configured to open and close the vent passage 7 to switch permission and inhibition of fluid movement through the vent passage 7. In the present embodiment, when the cassette 20 is attached to the apparatus body 10, the vent valve 25 is connected to the vent passage 7 in the cassette body 21.

The apparatus body 10 is provided with an aspiration pump 27. The aspiration pump 27 is configured to exert aspiration pressure to aspirate the eliminated tissue from the patient's eye E. Various configurations may be adopted for the aspiration pump 27. One example is a peristaltic pump to generate the aspiration pressure by pressing and rotating the flexible aspiration passage 6. As another example, a venturi pump to generate low pressure by feeding pressurized air into a venturi tube may be adopted. Further alternatively, several types of aspiration pumps 27 may be used for the ophthalmic surgical apparatus 1. In the present embodiment, when the cassette 20 is attached to the apparatus body 10, the aspiration pump 27 is connected to the aspiration passage 6B in the cassette body 21. To be specific, the aspiration pump 27 of the present embodiment is connected to the aspiration passage 6B downstream of the vent passage 7 and an aspiration pressure sensor 35 (which will be explained in detail below).

The cassette body 21 of the cassette 20 includes the non-positive displacement pump (which is sometimes called as a turbo-pump) 50. The non-positive displacement pump 50 is provided in the perfusion passage 5 (specifically, in the perfusion passage 5B provided in the cassette body 21). The ophthalmic surgical apparatus 1 is enabled to control pressure of the perfusion solution which will be supplied into the patient's eye E by use of the pump 50. Specifically, the pump 50 of the present embodiment is provided in the perfusion passage 5B on an upstream side of a portion where the vent passage 7 branches off. Accordingly, the ophthalmic surgical apparatus 1 is enabled to control pressure of the perfusion solution in the vent passage 7 by the pump 50 even when the vent passage 7 is open. A detailed explanation of the non-positive displacement pump 50 will be given below.

The apparatus body 10 is provided with the perfusion pressurizing motor 51. This perfusion pressurizing motor 51 is configured to rotate a rotor 58 (which will be explained in detail below) provided in the non-positive displacement pump 50 to exert pressure on the perfusion solution. In the present embodiment, when the cassette 20 is attached to the apparatus body 10, the motor 51 is connected to the rotor 58 provided in the non-positive displacement pump 50 in the cassette body 21. The motor 51 and the rotor 58 may be connected directly or connected via a gear or the like.

The apparatus body 10 is provided with the perfusion pressure sensor 34. The perfusion pressure sensor 34 is configured to detect pressure of the perfusion solution that is to be supplied from the surgical instrument into the eye. The perfusion pressure sensor 34 of the present embodiment is connected to the perfusion passage 5 on the downstream side of the rotor 58. As one example, the sensor 34 of the present embodiment is connected to the non-positive displacement pump 50 downstream of the rotor 58. Alternatively, the sensor 34 may be connected to the perfusion passage 5 downstream of the pump 50. For example, the sensor 34 may be connected to any one of the perfusion passage 5B in the cassette body 21, the perfusion passage 5C connected to the surgical instrument, and a perfusion passage inside the surgical instrument. In the present embodiment, when the cassette 20 is attached to the apparatus body 10, the sensor 34 is connected to the pump 50 in the cassette body 21.

The apparatus body 10 is provided with an aspiration pressure sensor 35. The aspiration pressure sensor 35 is configured to detect pressure of the fluid inside the aspiration passage 6. As one example, the controller 40 is configured to determine whether or not the aspiration passage 6 is occluded based on a detection result of pressure detected by the aspiration pressure sensor 35. The controller 40 is further configured to estimate pressure inside the patient's eye E (intraocular pressure) from the perfusion pressure detected by the perfusion pressure sensor 34 and the aspiration pressure detected by the aspiration pressure sensor 35. In the present embodiment, when the cassette 20 is attached to the apparatus body 10, the aspiration pressure sensor 35 is connected to the aspiration passage 6B in the cassette body 21.

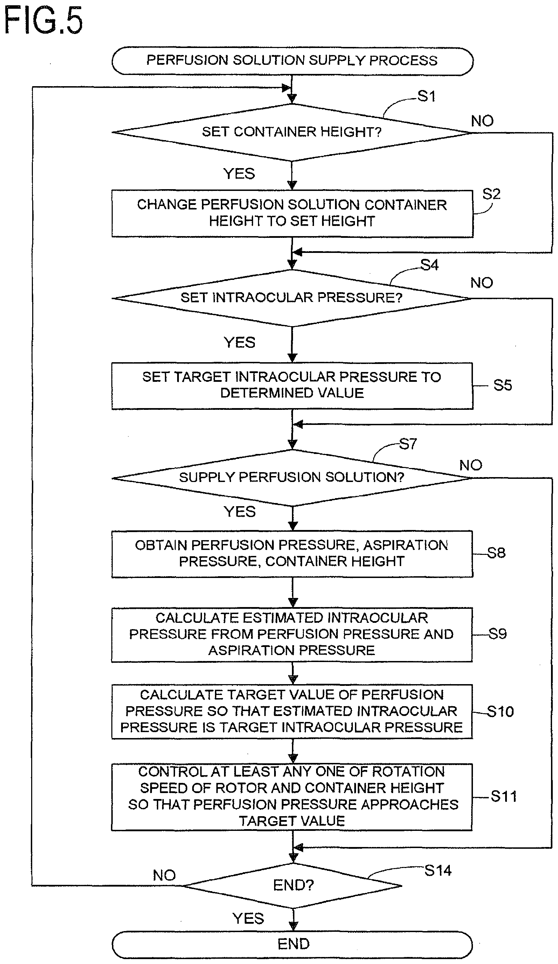

The apparatus body 10 is provided with the controller 40. The controller 40 is electrically connected with various configurations of the ophthalmic surgical apparatus 1 (including the monitor 11, the touch screen 12, the foot switch 18, the perfusion valve 24, the vent valve 25, the aspiration pump 27, the pole vertical-motion motor 29, the perfusion pressure sensor 34, and the aspiration pressure sensor 35). The controller 40 of the present embodiment is provided with a CPU (processor) 41, an ROM 42, an RAM 43, and a non-volatile memory (NVM) 44. The CPU 41 is in charge of controlling each section of the ophthalmic surgical apparatus 1. The ROM 42 is stored with various programs, initial values, and others. The RAM 43 temporarily stores various information. The non-volatile memory 44 is a non-fugitive storage medium which can retain storage contents even when supply of power source is shut off. The non-volatile memory 44 may be embodied with, for example, a hard disk drive, a flash ROM, a detachable USB memory, and the like. In the present embodiment, the non-volatile memory 44 is stored with a perfusion supply program and others to carry out a perfusion solution supply process (see FIG. 5) which will be mentioned below.

### <Non-positive displacement pump>

A configuration of the non-positive displacement pump 50 of the present embodiment is explained with reference to FIGs. 3 and 4. The non-positive displacement pump is a pump enabling to pressurize fluid without shutting off a passage of the fluid. In other words, the non-positive displacement pump does not need to have a space to contain the fluid in a hermetically closed state, and thus the fluid continuously flows irrespective of a rotating state of a rotor. The rotor does not block the perfusion passage 5, and the fluid pressure changes according to rotation speed of the rotor.

Types of the non-positive displacement pump include a centrifugal pump, a mixed flow pump, and an axial flow pump. In the centrifugal pump, the pressure that the rotor exerts on the fluid inside the pump is applied in a direction relative to a plane surface orthogonal to an axis of the rotor. In the mixed flow pump, the pressure that the rotor exerts on the fluid is applied in a direction relative to a conical surface, an axis of which is coaxial with the rotor axis. In the axial flow pump, the pressure that the rotor exerts on the fluid is applied in a direction relative to a cylindrical surface, an axis of which is coaxial with the rotor axis. The present embodiment is explained by employing a centrifugal pump as an example of the non-positive displacement pump 50. Other types of non-positive displacement pumps may also be adopted.

As shown in FIG. 3, the non-positive displacement pump 50 of the present embodiment includes a casing 54 and a cover 60. The casing 54 is provided with a cylindrical side wall and a bottom wall to close a bottom part of the side wall. The cover 60 closes an upper part of the casing 54. The casing 54 and the cover 60 constitute a pump chamber 55. The pump chamber 55 is provided inside with a blade-like rotor (an impeller in the present embodiment) 58 in a rotatable manner. The rotor 58 of the present embodiment includes a plurality of blades extending outwardly from a rotary shaft 59, and rotation of the blades applies pressure to the fluid inside the pump chamber 55. The rotary shaft 59 of the rotor 58 extending in a vertical direction penetrates the bottom wall of the casing 54 and protrudes downward. When the cassette 20 (see FIGs. 1 and 2) is attached to the apparatus body 10, a lower end of the rotary shaft 59 of the rotor 58 is joined to a rotary shaft 52 of the perfusion pressurizing motor 51.

The casing 54 is provided with a protruding part 56 protruding outward from a cylindrical part of the casing 54. The cover 60 includes an inlet port 61 positioned far away from the protruding part 56 to bring the perfusion solution supplied from the perfusion solution container 17 into the pump chamber 55 through the perfusion passage 5. The cover 60 further includes an outlet port 62 positioned corresponding to the protruding part 56 to bring the perfusion solution into the surgical instrument from the pump chamber 55.

As shown in FIGs. 3 and 4, an outer circumferential end of each blade of the rotor 58 is separated from the side wall of the casing 54. Namely, there is a clearance between the rotor 58 and the side wall of the casing 54. The non-positive displacement pump 50 is provided with the inlet port 61 to flow the perfusion solution into the pump chamber 55 and the outlet port 62 to discharge the perfusion solution stored in the pump chamber 55. The rotor 58 is placed in a predetermined position in the pump chamber 55 so as not to interrupt the perfusion passage of the perfusion solution which flows from the inlet port 61 to the outlet port 62 through the pump chamber 55. Accordingly, the perfusion solution flowing from the inlet port 61 to the pump chamber 55 is allowed to be discharged out of the outlet port 62 irrespective of the state of the rotor 58. Rotation of the rotor 58 exerts a centrifugal force to apply pressure to the perfusion solution that has flown in the pump chamber 55 from the inlet port 61. The thus pressurized perfusion solution is moved to the protruding part 56 and discharged out of the outlet port 62. Herein, the non-positive displacement pump 50 may further be provided with a guiding blade at a circumference of the rotor 58 to guide flow of the perfusion solution which is pressurized by the rotor 58.

In the present embodiment, the outlet port 62 is provided upward than the blades of the rotor 58. Inside the pump chamber 55 is filled with the perfusion solution by this configuration, and thus air is less possibly entered in the pump chamber 55 during surgery. Accordingly, there is less possibility of intrusion of air into the eye from the pump chamber 55. Even if the outlet port 62 is provided in a position equal to or lower than the blades of the rotor 58, the non-positive displacement pump 50 is enabled to apply pressure to the perfusion solution.

As shown in FIG. 3, a sensor connecting part 64 to which the perfusion pressure sensor 34 (see FIG. 2) is connected is provided in a position corresponding to the protruding part 56 of the casing 54. The perfusion pressure sensor 34 of the present embodiment is therefore connected to the passage downstream of the rotor 58. As a result, the perfusion pressure sensor 34 can appropriately detect pressure of the perfusion solution that is pressurized by the non-positive displacement pump 50.

### <Perfusion solution supply process>

A perfusion solution supply process performed by the ophthalmic surgical apparatus 1 of the present embodiment is explained with reference to FIG. 5. The perfusion solution supply process is a process for controlling supply of the perfusion solution into an eye. When supply of the perfusion solution is allowed, the CPU 41 carries out the perfusion solution supply process illustrated in FIG. 5 according to a perfusion solution supply program.

Firstly, the CPU 41 determines whether or not a command of setting the height of the perfusion solution container 17 is input by the user (S1). In the present embodiment, the user operates the touch screen 12 to input the operation command of setting the height of the perfusion solution container 17. When the height is not set (S1:NO), the process proceeds to a step S4. When the height is set (S1:YES), the CPU 41 controls the drive to the pole vertical-motion motor 29 to change the height of the perfusion solution container 17 held by the container holding unit 16 to the determined height (S2).

Subsequently, the CPU 41 determines whether or not a command of setting the intraocular pressure of the patient's eye E is input (S4). In the present embodiment, the user operates the touch screen 12 to input the operation command of setting the intraocular pressure in the ophthalmic surgical apparatus 1. When the intraocular pressure is not set (S4:NO), the process succeeds to a step S7. When the intraocular pressure is set (S4:YES), the CPU 41 sets a target intraocular pressure to be the value input by the user (S5).

Subsequently, the CPU 41 determines whether or not an operation by the user to supply the perfusion solution is performed (S7). In the present embodiment, the user inputs the operation command of carrying out supply of the perfusion solution into the eye in the ophthalmic surgical apparatus 1 by the foot switch 18. When the foot switch 18 is not operated (S7:NO), the process succeeds to a step S 14.

When the foot switch 18 is operated (S7:YES), the CPU 41 obtains the perfusion pressure, the aspiration pressure, and the height of the perfusion solution container 17 (S8). In the present embodiment, the CPU 41 obtains the perfusion pressure from the perfusion pressure sensor 34 (see FIG. 2) and obtains the aspiration pressure from the aspiration pressure sensor 35 (see FIG. 2). As one example, the CPU 41 further obtains the height of the perfusion solution container 17 from an encoder (not-shown) provided in the pole vertical-motion motor 29. A method of obtaining the height of the perfusion solution container 17 may be modified. For example, a sensor to detect the height of the perfusion solution container 17 may be provided in the container holding unit 16.

The CPU 41 calculates an estimated intraocular pressure of the patient's eye E from the obtained values of the perfusion pressure and the aspiration pressure (S9). The perfusion pressure and the aspiration pressure have much influence on the intraocular pressure. The CPU 41 can thus calculate the estimated intraocular pressure value from the perfusion pressure and the aspiration pressure. Subsequently, the CPU 41 calculates a target value of the perfusion pressure so that the estimated intraocular pressure approaches the target intraocular pressure (S10). The CPU 41 controls at least any one of the rotation speed of the rotor 58 and the height of the perfusion solution container 17 to make the perfusion pressure detected by the perfusion pressure sensor 34 approach the target value calculated in the step S10 (S11).

The CPU 41 determines whether or not a command of terminating supply of the perfusion solution is input (S14). When the command of termination is not input (S14:NO), the process returns to the step S1. When the termination command is input (S14:YES), the perfusion solution supply process is ended.

As explained above, the ophthalmic surgical apparatus 1 of the present embodiment is provided with the non-positive displacement pump 50 in the perfusion passage 5. The non-positive displacement pump 50 is configured such that the perfusion passage 5 is not shut off by rotation of the rotor 58 (the perfusion passage 5 is not blocked). In other words, the rotor 58 of the non-positive displacement pump 50 does not create a space for hermetically containing the perfusion solution. Accordingly, the ophthalmic surgical apparatus 1 is enabled to continuously feed the perfusion solution into the eye and to control the pressure of the perfusion solution supplied into the eye by use of the non-positive displacement pump 50. Further, the non-positive displacement pump 50 can directly apply pressure to the continuously fed perfusion solution with no need of providing compressed air or the like. Thus, speed to change the perfusion pressure is increased (responsiveness is good). Types of the perfusion solution container 17 are not limited. The apparatus configuration can be easily simplified. Moreover, depending on the height of the perfusion solution container 17, the perfusion solution is kept to be supplied into the eye even when the non-positive displacement pump 50 or the perfusion pressurizing motor 51 is broken. It is hence easy to prevent any risks caused by breakage. Therefore, the ophthalmic surgical apparatus 1 of the present embodiment can supply the perfusion solution into the eye in an appropriate manner.

In the ophthalmic surgical apparatus 1 of the present embodiment, the non-positive displacement pump 50 is provided in the cassette 20. The perfusion pressurizing motor 51 is connected to the rotor 58 by the attachment of the cassette 20 to the apparatus body 10. In this example, the user detaches the used cassette 20 and attaches a new cassette 20 to the apparatus body 10, so that the non-positive displacement pump 50 through which the perfusion solution passes can be easily kept clean. Further, the perfusion pressurizing motor 51 and others are not provided in the cassette 20, thereby easily reducing the cost for consumable cassettes 20.

The ophthalmic surgical apparatus 1 of the present embodiment is provided with the container holding unit 16 enabling to hold the perfusion solution container 17 at a position equal to or higher than the height of the patient's eye E. The perfusion solution contained in the container 17 is hence let flown through the non-positive displacement pump 50 and supplied into the eye by at least any one of the motive power of the displacement pump 50 and the gravity force. Accordingly, when at least one of the non-positive displacement pump 50 and the perfusion pressurizing motor 51 is broken, for example, the perfusion solution can be supplied into the eye by its gravity force. As a result, the risk caused by breakage and others is prevented. Further, the user can appropriately choose a method of supplying the perfusion solution into the eye according to a type of surgery or the like.

In the present embodiment, the height of the perfusion solution container 17 is changed by the pole vertical-motion motor 29. When the height of the perfusion solution container 17 is changed, the perfusion pressure applied by the gravity force is changed. Therefore, the ophthalmic surgical apparatus 1 of the present embodiment enables to control the perfusion pressure by further various methods. Even when at least one of the non-positive displacement pump 50 and the perfusion pressurizing motor 51 is broken, for example, the perfusion pressure can be adjusted by changing the height of the container 17 by the pole vertical-motion motor 29. The ophthalmic surgical apparatus 1 is further enabled to change the height of the container 17 and thus to change the ratio of the non-positive displacement pump 50 and the gravity force each contributing to the perfusion pressure.

The ophthalmic surgical apparatus 1 of the present embodiment is enabled to control the rotation speed of the rotor 58 based on the perfusion pressure which is detected by the perfusion pressure sensor 34 so that the perfusion pressure approaches the target value. Accordingly, the non-positive displacement pump 50 having good responsivity can control the perfusion pressure more accurately.

The technique disclosed in the above embodiment is merely an example of the present disclosure, and the technique illustrated in the above embodiment may be modified. For example, the non-positive displacement pump 50 is provided in the disposable cassette 20 in the above embodiment, but the displacement pump 50 may not be provided in the cassette 20. As one alternative, the displacement pump 50 may be attached to the apparatus body 10 in a detachable manner. In this case, the user detaches the displacement pump 50 from the apparatus body 10 and performs sterilization and others, so that the displacement pump 50 can be kept clean.

Another modification is to change the configuration of the non-positive displacement pump 50. For example, the displacement pump 50 in the above embodiment has difficulty in aspirating the perfusion solution from the perfusion solution container 17 when the container 17 is placed in a low position. However, it is possible to adopt a non-positive displacement pump which is able to aspirate solution from a low position. Further, as mentioned above, a mixed flow pump, an axial flow pump, and others may be adopted.

The ophthalmic surgical apparatus 1 of the above embodiment is configured to automatically change the height of the perfusion solution container 17 by the pole vertical-motion motor 29. Alternatively, in another case that the user manually changes the height of the container 17, at least a part of the technique illustrated in the above embodiment may be employed.

In the above embodiment, an estimated intraocular pressure of the patient's eye E is calculated from the perfusion pressure detected by the perfusion pressure sensor 34 and the aspiration pressure detected by the aspiration pressure sensor 35. The target value of the perfusion pressure is calculated such that the calculated estimated intraocular pressure becomes the target intraocular pressure. As one alternative for this, for example, the ophthalmic surgical apparatus 1 may be configured to obtain the detected result from an intraocular pressure sensor for detecting the intraocular pressure of the patient's eye E and to calculate the target value of the perfusion pressure such that the obtained intraocular pressure becomes the target intraocular pressure. In this case, the intraocular pressure of the patient's eye E is further accurately adjusted. The intraocular pressure sensor may be, for example, a pressure sensor which is directly inserted into the patient's eye E. Further, in the above embodiment, the perfusion pressure sensor 34 is provided in the apparatus body 10. As alternative for this, the position of the pressure sensor may be changed. For example, the pressure sensor may be provided in a part (such as a perfusion passage inside the handpiece) of a surgical instrument (such as a handpiece) which is to be inserted into the eye.

## Claims

1. An ophthalmic surgical apparatus (1) configured to supply perfusion solution into a patient's eye (E) from a perfusion solution container (17) stored with the perfusion solution, the ophthalmic surgical apparatus (1) comprising:
a perfusion passage (5) configured to introduce the perfusion solution in the perfusion solution container (17) to a surgical instrument (30,31) which will be inserted in the eye;
a non-positive displacement pump (50) provided in the perfusion passage (5), the non-positive displacement pump (50) including a blade-like rotor (58) which is rotatable without shutting off the perfusion passage;
a motor (51) configured to rotate the rotor (58) and thus to apply pressure to the perfusion solution; and
a controller (40) configured to control the drive to the motor (51).

2. The ophthalmic surgical apparatus (1) according to claim 1, **characterized in that** the ophthalmic surgical apparatus further comprises:
an apparatus body (10) which is at least provided with the motor (51); and
a cassette (20) including at least a part of the perfusion passage (5) and the non-positive displacement pump (50), the cassette being configured to be attached to the apparatus body (10) in a detachable manner, and
the motor (51) is configured to be connected with the rotor (58) by attachment of the cassette (20) to the apparatus body (10).

3. The ophthalmic surgical apparatus (1) according to claim 1 or 2 further comprising a container holding unit (16) configured to hold the perfusion solution container (17) at a position equal to or higher than a height of the patient's eye (E).

4. The ophthalmic surgical apparatus (1) according to claim 3, **characterized in that** the ophthalmic surgical apparatus further includes a height changing part (29) to change the height of the perfusion solution container (17) which is held by the container holding unit (16).

5. The ophthalmic surgical apparatus (1) according to any of claims 1 to 4, **characterized in that**
the ophthalmic surgical apparatus further includes a pressure detection part (34,35) configured to detect at least any one of pressure of the perfusion solution, which is supplied from the surgical instrument (30,31) into the eye, and an intraocular pressure of the patient's eye, and
the controller (40) is configured to control a rotation speed of the rotor (58) based on pressure which is detected by the pressure detection part (34,35) so that the pressure of the perfusion solution which is to be supplied into the eye is made to approach a target pressure value.

6. The ophthalmic surgical apparatus (1) according to any one of claims 1 to 5, **characterized in that**
the non-positive displacement pump (50) is provided with an inlet port (61) to flow the perfusion solution into a pump chamber (55) and an outlet port (62) to discharge the perfusion solution stored in the pump chamber (55), and
the rotor (58) is provided in a predetermined position inside the pump chamber (55) without shutting off the perfusion passage (5) of the perfusion solution which flows from the inlet port (61) to the outlet port (62) via the pump chamber (55).
